# EUROPÄISCHE PATENTSCHRIFT

(11) **EP 0 780 370 B1**
(45) Veröffentlichungstag und Bekanntmachung des Hinweises auf die Patenterteilung: **05.02.2003**
(21) Anmeldenummer: 96117124.6
(22) Anmeldetag: 25.10.1996
(51) Int. Cl.: C07C 323/58, C07C 319/28

(54) **Verfahren zur Herstellung von D,L-Methionin oder dessen Alkalisalzen**
Process for the preparation of D,L-methionine or alkali salts thereof
Procédé de préparation de la D,L-méthionine ou ces sels alkalins

(30) Priorität: 18.12.1995 DE 19547236
(43) Veröffentlichungstag der Anmeldung: 25.06.1997
(60) Teilanmeldung: 00127621.1
(73) Patentinhaber: Degussa AG, 40474 Düsseldorf (DE)
(72) Erfinder: Geiger, Friedhelm, Dr., 63526 Erlensee (DE); Halsberghe, Baudouin, Mobile, Alabama (US); Hasselbach, Hans-Joachim, Dr., 63571 Gelnhausen (DE); Hentschel, Klaus, Dr., 63517 Rodenbach (DE); Huthmacher, Klaus, Dr., 63571 Gelnhausen (DE); Körfer, Martin, 63867 Johannesberg (DE); Mannsfeld, Sven-Peter, Dr., Mobile, Alabama (US); Tanner, Herbert, Dr., 63457 Hanau (DE); Theissen, Ferdinand, Dr., 53332 Bornheim (DE); Vanrobaeys, Jose, Mobile, Alabama (US); Willigerodt, Klaus, Dr., Mobile, Alabama (US)

(56) Entgegenhaltungen:
- EP-A- 0 143 218
- DE-A- 1 620 332
- DE-A- 1 906 405
- GB-A- 1 186 538
- NL-A- 7 311 018
- CHEMICAL ABSTRACTS, vol. 120, no. 13, 28.März 1994 Columbus, Ohio, US; abstract no. 164902t, TERASAWA T. ET AL.: "Preparation of methionine" Seite 1264; Spalte 2; XP002029147 & JP 05 286 926 A (SUMITOMO CHEMICAL CO.) 2.November 1993
- CHEMICAL ABSTRACTS, vol. 84, no. 7, 16.Februar 1976 Columbus, Ohio, US; abstract no. 44666k, TAKAGI K. ET AL.: ".alpha.-Amino acids" Seite 556; Spalte 2; XP002033766 & JP 50 106 901 A (SUMITOMO CHEMICAL CO., LTD.) 22.August 1975
- CHEMICAL ABSTRACTS, vol. 85, no. 7, 16.August 1976 Columbus, Ohio, US; abstract no. 47049t, KOJIMA T. ET AL.: "DL-Methionine alkali salts" Seite 577; Spalte 1; XP002033767 & JP 50 157 318 A (KANEBO LTD.) 19.Dezember 1975
- DATABASE WPI Section Ch, Week 9122 Derwent Publications Ltd., London, GB; Class A41, AN 91-159964 XP002033769 MIZUNO T. ET AL.: "Production of alpha-amino acid" & JP 03 095 145 A (SUMITOMO CHEM IND KK) , 19.April 1991
- DATABASE WPI Section Ch, Week 9122 Derwent Publications Ltd., London, GB; Class A41, AN 91-159965 XP002033770 MIZUNO T. ET AL.: "Production of alpha-amino acid" & JP 03 095 146 A (SUMITOMO CHEM IND KK) , 19.April 1991
- CHEMICAL ABSTRACTS, vol. 117, no. 17, 26.Oktober 1992 Columbus, Ohio, US; abstract no. 172112p, MIZUNO T. ET AL.: "Preparation of methionine" Seite 914; Spalte 1; XP002033768 & JP 04 169 570 A (SUMITOMO CHEMICAL CO., LTD.) 17.Juni 1992
- DATABASE WPI Section Ch, Derwent Publications Ltd., London, GB; Class A25, AN 73-64461U XP002033771 & JP 48 014 618 A (KANEGAFUCHI CHEMICAL INDUSTRIES CO., LTD.) , 23.Februar 1973
- DATABASE WPI Section Ch, Derwent Publications Ltd., London, GB; Class A25, AN 73-64462U XP002033772 & JP 48 014 619 A (KANEGAFUCHI CHEMICAL INDUSTRIES CO., LTD.) , 23.Februar 1973

## Beschreibung

Die Erfindung betrifft ein Verfahren zur Herstellung von D,L-Methionin oder einem Salz des D,L-Methionins durch Hydrolyse von 5-(2-Methylmercaptoethyl)-hydantoin in Apparaturen mit Einbauten aus metallischem Zirkonium oder Zirkoniumlegierung.

Die Syntheseschritte lassen sich durch folgende Reaktionsgleichungen veranschaulichen:
5- (2-Methylmercapto)-hydantoinbildung (nicht gegenhand der Erfindung) 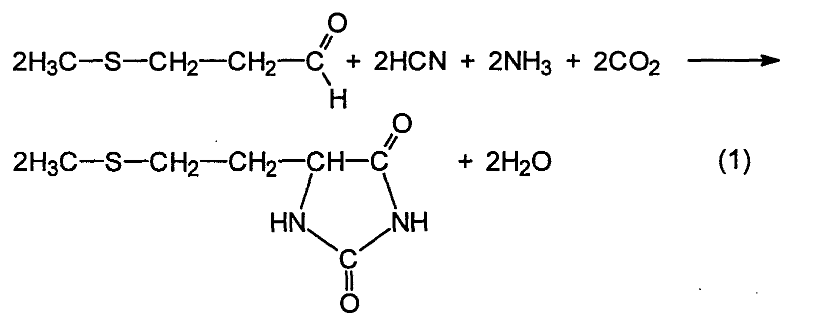 Salzbildung des D,L-Methionins: 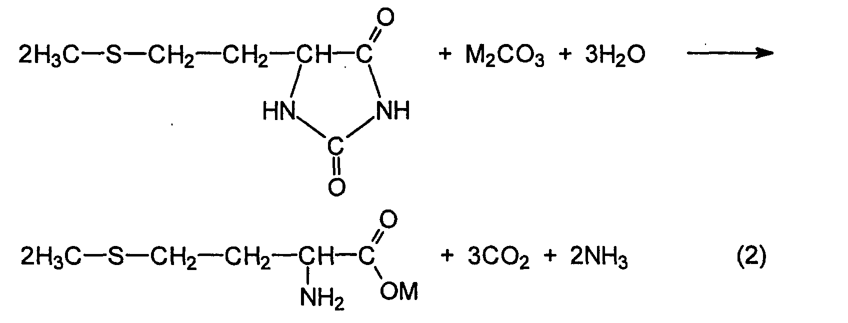 D,L-Methionin-Freisetzung: 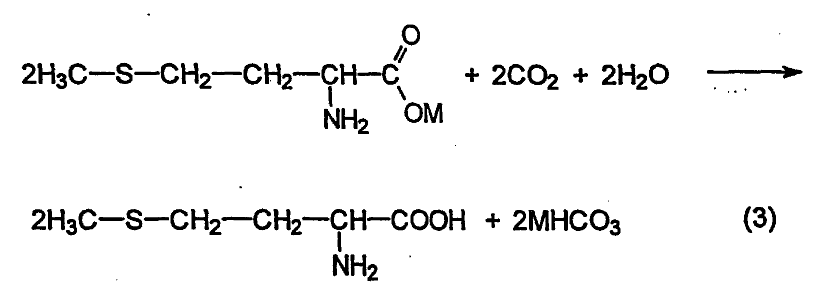 M steht für Alkali, Ammonium, insbesondere Kalium.

Die Verfahrensstufen 5-(2-Methylmercaptoethyl)-hydantoin-Bildung, Methioninat-Bildung sowie Methionin-Freisetzung können für sich vorteilhaft kontinuierlich betrieben werden und werden zweckmäßigerweise hintereinander verschaltet in einen insbesondere gesamtkontinuierlich verlaufenden Prpzeß integriert.

Besonders vorteilhaft werden die Komponenten Ammoniak und Kohlendioxid entsprechend den Prozeßmöglichkeiten recycliert, d. h. in die zuvor ablaufenden Verfahrensstufen wieder eingesetzt. Insbesondere bei der Verwendung von Kalium werden möglichst die gesamten Alkali enthaltenden Agentien in den Prozeß zurückgeführt.

Die 5-(2-Methylmercaptoethyl)-hydantoinbildung ist grundsätzlich bekannt. Generell wird dabei entweder von den oben unter 1) beschriebenen Komponenten ausgegangen oder von solchen Komponenten, aus denen diese Komponenten herstellbar sind.

Die alkalische Hydrolyse von 5-(2-Methylmercaptoethyl)-hydantoin ist seit langem bekannt. So beschreiben die US-A-2,527,366 und US-A-2,557,913 die Hydrolyse von 5-(2-Methylmercaptoethyl)-hydantoin in einer wäßrigen Bariumhydroxidlösung unter Druck und erhöhter Temperatur. Diese Verfahren erfordern aber erhebliche Mengen an teurem Bariumhydroxid, außerdem muß das Barium als Neutralsalz wieder abgetrennt werden.

Aus der US-A-2,557,920 ist bekannt, daß α-Aminosäuren durch Verseifung von Hydantoinen unter Verwendung von Natriumhydroxid entstehen. Bei diesen Verfahren werden aber mindestens 3 Mol Natriumhydroxid je Mol Hydantoin benötigt. Analog verhält es sich beim Einsatz von Kaliumhydroxid.

Ferner ist aus der US-A-4,272,631 bekannt, daß ein Gemisch aus Alkali- und Erdalkalihydroxid zur Verseifung von 5-(2-Methylmercaptoethyl)-hydantoin verwendet werden kann. Allerdings müssen bei diesen Verfahren die Erdalkaliionen bei der Freisetzung von Methionin erst abgetrennt werden, so daß nur Ausbeuten von max. 80,5 % erreicht werden.

In der US-A-4,259,925 wird die Hydrolyse von 5-(2-Methylmercaptoethyl)-hydantoin unter Druck bei 105 bis 230 °C in einem Medium, das ein Metallhydroxid und einen Alkohol mit einem Siedepunkt von 125 bis 130 °C enthält, durchgeführt. Nachteilig ist, daß der hochsiedende Alkohol wieder zurückgewonnen werden muß. Außerdem beträgt die Ausbeute nur 65 %.

In der DE-C-19 06 405 wird die Hydrolyse von 5- (2-Methylmercaptoethyl) -hydantoin unter Verwendung einer wässrigen Lösung von Alkalicarbonat und/oder Alkalihydrogencarbonat beschrieben. Während der Hydrolyse werden Ammoniak und Kohlendioxid laufend entfernt. Von den Alkalicarbonaten wird Kaliumcarbonat bevorzugt; es wird ein Mol-Verhältnis Hydantoin zu Alkali von 1 : 1 bis 1 : 5 angewendet. Die Hydrolyse wird unter Druck bei 120 bis 220 °C durchgeführt. Die kontinuierliche Druckapparatur besteht aus drei aufwendig hintereinander geschalteten Umlaufverdampfern. Die Alkalimethioninatlösung wird zur Freisetzung von D,L-Methionin mit Kohlendioxid verwendet; die Mutterlauge aus der Abtrennung des auskristallisierten Methionins wird im Kreislauf ggf. mit Ausschleusung von 1 - 2 % wieder zur Hydrolyse des Hydantoins eingesetzt.

Die DE-B-15 18 339 beschreibt ein Verfahren, bei dem bei der Hydrolyse entstandene gasförmige Reaktionsprodukte (Ammoniak und Kohlendioxid) aus der Reaktion entfernt werden, um das Reaktionsgleichgewicht in Richtung der Aminosäure zu verschieben, wodurch die Ausbeute gesteigert wird. Um dies zu erreichen, ist jedoch eine komplexe apparative Anordnung zur Druckregelung der Gasströme erforderlich.

Die japanische Patentschrift 49/116 008 beschreibt ein Verfahren, bei dem die Hydrolyse von 5-(2-Methylmercaptoethyl)-hydantoin in Gegenwart von Vanadinsäuren, Molybdänsäuren, Wolframsäuren oder deren Derivate durchgeführt wird. Die Ausbeuten betragen ca. 70 %. Schwierig ist die Abtrennung des Katalysators. Zur Herstellung einer hochkonzentrierten Methionin-haltigen Lösung muß Alkali, z. B. eine Kaliumverbindung, zugesetzt werden.

Aus der japanischen Patentanmeldung 75/106 901 (C. A. 84, 44666k (1976)) ist bekannt, daß Methionin durch Hydrolyse von 5-(2-Methylmercaptoethyl)-hydantoin in Gegenwart von etwa 1,2 eq Natriumhydroxid und von etwa 9 eq Ammoniak bei 180 °C hergestellt wird. Die bei dieser Arbeitsweise intermediär entstehende Natriummethioninat-Lösung enthält jedoch zwangsläufig neben dem Natriummethioninat auch Natriumcarbonat, das bei dieser Reaktionsführung ausfällt und deshalb insbesondere bei einem kontinuierlichen Prozeß störend ist. Analoges gilt bei der Verwendung von Kaliumhydroxid sowie für die Verfahrensweise gemäß DE-C 19 06 405.

Die DE 26 14 411 A beschreibt die Hydrolyse von 5-(2-Methylmercaptoethyl)-hydantoin mit Wasser in Gegenwart von Imidazol bei 160 °C. Die Ausbeuten sind niedrig und auch hier muß eine Alkaliverbindung zur Erzeugung einer hohen Lösungskonzentration zugegeben werden.

Die japanischen Patentanmeldungen JP 03/95145 und JP 03/95146 beschreiben die Hydrolyse von Hydantoinen mit Wasser bei erhöhter Temperatur und erhöhtem Druck in Gegenwart von Metalloxiden oder -oxidgemischen, wie z. B. ZrO₂, TiO₂, Nb₂O₅ oder TiO₂-Nb₂O₅; die Ausbeuten liegen allerdings nur bei 65 bis 66 %. Diese Lösungen müssen mit einer Alkaliverbindung neutralisiert werden.

All diese Verfahren bringen entweder geringe Ausbeuten oder haben den Nachteil, daß Methionin oder Salze wie Carbonate während des Prozesses ausfallen, wodurch weitere Verfahrensschritte verursacht werden und insbesondere großtechnische Verfahren, ganz besonders kontinuierliche Verfahren, kaum möglich sind.

Die Freisetzung des Methionins aus dem Alkalisalz ist allgemein bekannt. Nach dem Prinzip, starke Säuren setzen schwächere Säuren aus ihren Salzen frei, wird z. B. mit Salzsäure, Schwefelsäure, Phosphorsäure oder einem stark sauren Ionenaustauscher freies D,L-Methionin ausgefällt (DE-C-21 40 506; DE-C-21 22 491; DE-A-29 12 066; BE-A-877 200, US-A-3 433 832, FR-A-1 532 723). Allerdings muß dann noch das als Nebenprodukt anfallende Alkalisalz abgetrennt werden. Da die eingesetzte Säure im allgemeinen nicht mehr zurückgewonnen wird, ist diese Vorgehensweise für ein kontinuierliches, wirtschaftliches und umweltschonendes Herstellverfahren ungeeignet.

Zweckmäßigerweise wurde deshalb - wie beispielsweise in den Patentschriften US-A-2 557 913, DE-C-19 06 405 und JP 42/44056 beschrieben - D,L-Methionin aus den Hydrolyselösungen des 5-(2-Methylmercaptoethyl)-hydantoins mit Kohlendioxid in wässriger Lösung ausgefällt. Bei dieser Methode fällt i. d. R. das D,L-Methionin in Form von dünnen Plättchen oder schuppenförmig an. Dies bedingt eine schlechte Filtrierbarkeit des Produkts und behindert ein Auswaschen des Kristallkuchens; außerdem fällt ein D,L-Methionin mit schlechtem Fließverhalten und Neigung zur Verklumpung an. Um diesen Nachteilen entgegenzuwirken, werden gemäß der japanischen Patentschrift JP 42/44056 Zusätze wie Kasein oder wasserlösliche, hochmolekulare Cellulosederivate bei der Methioninfällung mit Kohlendioxid zugesetzt.

Aufgabe der vorliegenden Erfindung ist, ein Verfahren zur Herstellung von Methionin oder eines Alkalisalaes des Methionins durch Hydrolyse von 5-(2-Methylmercaptoethyl)-hydantoin in hoher Ausbente bereitzustellen.

Gegenstand der Erfindung ist ein Verfahren zur Herstellung eines Alkalisalzes von Methionin durch Hydrolyse von 5-(2-Methylmercaptoethyl)-hydantoin in Gegenwart einer wässrigen Lösung enthaltend Alkali und Kohlendioxid, und ggf. weitere Umsetzung zum Methionin, wobei die Hydrolyse in Apparaturen mit Einbauten aus metallischem Zirkonium oder einer Zirkonium-Legierung, vorteil haft enthaltend mind. 10 Gew.-% Zirkonium), durchgeführt wird. Vorteil haft ist, daß zumindest zu Beginn der Hydrolyse in Gegenwart von mind. 0,1 eq, insbesondere bis 7 eq, Ammoniak je Equivalent 5-(2-Methylmercaptoethyl)-hydantoin gearbeitet wird.

Es hat sich gezeigt, daß es besonders vorteilhaft ist, wenn die Hydrolyse von Anfang an in Gegenwart von Alkali und Kohlendioxid, d. h., daß insbesondere eine Mischung von Alkaliverbindungen vorliegt, insbesondere Alkalihydrogencarbonat, Alkalicarbonat, Alkalihydroxid, wobei Alkali insbesondere für Kalium und Natrium steht, durchgeführt wird. Die Menge von Alkali und Kohlendioxid ist dabei zweckmäßig wenigstens die auf das Hydantoin bezogene stöchiometrische Menge. Diese kann nach oben deutlich überschritten werden. Ein Molverhältnis mit einem Überschuß von etwa 3 : 1 bezogen auf das Hydantoin ist besonders vorteilig; grundsätzlich ist davon auszugehen, daß ein noch größerer Überschuß noch günstiger ist. Für die Praxis jedoch sind Verhältnisse von 1,5 : 1 - 2 : 1 besonders bevorzugt. Erfindungsgemäß wird dabei noch zusätzlich etwas Ammoniak zugegeben, der entsprechend ebenfalls teils auch in Form von Ammoniumverbindungen vorliegt. Besonders vorteilhaft ist es hierbei, wenn zu Beginn der Hydrolyse je Mol 5-(2-Methylmercaptoethyl)-hydantoin max. 7 Mol Ammoniak (incl. Ammoniumverbindungen) vorliegt. Hierdurch wird erreicht, daß die Hydrolyse praktisch ohne Nebenproduktbildung und in guten Ausbeuten abläuft und andererseits nicht oder nur wenig Alkalicarbonat ausfällt. Besonders vorteilhaft ist hierbei, wenn während der Hydrolyse Ammoniak und/oder Kohlendioxid, ggf. zusammen mit Wasser, aus dem Reaktionssystem ausgeschleust werden. Hierdurch lassen sich die Reaktionsbedingungen besonders günstig steuern, so daß weiterhin kein Alkalicarbonat ausfällt und die Reaktion vollständig verläuft. Die Hydrolyseapparatur enthalt Einbauten aus Zirkonium oder aus einer entsprechenden Zirkoniumlegierung ist. Es wurde festgestellt, daß das Zirkonium einen besonders günstigen, vermutlich katalytischen Effekt auf die Hydrolyse ausübt. Als günstiger Nebeneffekt ergibt sich hierbei, daß diese Apparate gut widerstandsfähig und somit langlebig sind, so daß der Einsatz von Zirkoniumapparaturen keine apparatebedingten Nachteile gegenüber anderen Apparaturen bringt.

Die Hydrolyseverfahren werden günstigerweise bei einer Temperatur von 120 bis 250 °C und entsprechend einem Druck von 5 bis 30 bar durchgeführt. In diesem Bereich ergeben sich sehr gute Umsetzungen und geringe Nebenproduktbildung. Vorteilhaft ist auch, wenn die Alkalikomponente zumindest equimolar bezüglich des 5-(2-Methylmercaptoethyl)-hydantoins eingesetzt wird. Man erhält dann neben der vollständigen Hydrolyse auch praktisch quantitativ das entsprechende Alkalisalz des Methionins. Bevorzugt enthält die Hydrolyselösung zu Beginn auch bereits Methionin bzw. dessen Salz, auch dies hat einen günstigen, vermutlich autokatalytischen Effekt auf die Hydrolyse.

Bei dieser Verfahrensweise kann vorteilhaft im Laufe bzw. nach der Hydrolyse praktisch das gesamte Ammoniak und das gesamte Kohlendioxid aus der Hydrolyselösung abgezogen werden, so daß das Hydrolysat im wesentlichen frei von Ammoniak und Kohlendioxid entnommen werden kann.

Auch hier ist es besonders vorteilhaft, das Verfahren kontinuierlich durchzuführen. Ganz besonders vorteilhaft ist hierbei, daß Kohlendioxid und Ammoniak recycliert werden können.

Im folgenden wird die Erfindung hinsichtlich der Hydantoin-Hydrolyse näher beschrieben, wobei einzelne nähere Spezifikationen grundsätzlich für sich allein auch für die oben gegebene allgemeine Verfahrensweise gelten. Die im folgenden wiedergegebene Verfahrensweise wird für entsprechende Kaliumverbindungen beschrieben, da es sich hierbei um die am meisten bevorzugte Ausführungsform handelt.

Erfindungsgemäß wird eine Kaliummethioninat-Lösung durch Hydrolyse von 5-(2-Methylmercaptoethyl)-hydantoin in Gegenwart von Kaliumhydroxid, Kaliumcarbonat und/oder Kaliumhydrogencarbonat oder eines Gemisches davon und bei Anwesenheit von überschüssigem Ammoniak, Kohlendioxid, Kohlensäure oder eines Gemisches davon in Wasser bei einer Temperatur von 120 bis 250 °C und einem Druck von 5 bis 30 bar erhalten. Vorteilhaft erfolgt die Hydrolyse des Hydantoins bezogen auf das Hydantoin in Gegenwart von 1 bis 15 eq einer oder mehrerer Kaliumverbindungen (z. B. KOH, KHCO₃, K₂CO₃, Kaliummethioninat). Während bzw. nach der Hydrolyse ist es außerdem vorteilhaft, wenn entstehendes oder noch vorhandenes Ammoniak und/oder Kohlendioxid ganz oder teilweise aus dem Reaktionssystem abgetrennt wird. Grundsätzlich kann jedes 5-(2-Methylmercaptoethyl)-hydantoin eingesetzt werden, günstigerweise wird ein Hydantoin eingesetzt, das wie oben beschrieben erhältlich ist.

Vorteilhaft ist das Ammoniak zu Beginn der Hydrolyse ein Mol-Verhältnis zu Kohlendioxid von 1,1 bis 8,0. Günstig ist auch ein Mol-Verhältnis des Ammoniaks zum Hydantoin von 0,2 bis 5. Bei der beschriebenen Verfahrensweise ist es möglich, das Ammoniak und das Kohlendioxid unmittelbar aus dem oben beschriebenen Verfahren, der Hydantoin-Herstellung, zu übernehmen, so daß das Hydantoin unmittelbar aus der Hydantoin-Herstellung zusammen mit dem noch verbliebenen Ammoniak und Kohlendioxid in die Hydrolyse eingebracht werden kann, wobei hier dann noch eventuell gewünschte andere Ammoniak- und/oder Kohlendioxidkonzentrationen eingestellt werden können.

Bei dem erfindungsgemäßen Verfahren findet die Hydrolyse des 5- (2-Methylmercaptoethyl)-hydantoins bei Temperaturen von 120 - 250 °C, vorzugsweise von 150 - 200 °C, insbesondere von 160 - 180 °C, statt; der Druck während der Reaktion soll 5 - 30 bar, vorzugsweise 5 - 10 bar, insbesondere 7 - 9 bar, betragen.

Das Verfahren wird vorteilhaft in einer mit Dampf beheizten Kolonne mit Einbauten durchgeführt, bei der die Innenwand und die Einbauten aus Zirkonium oder einer mind. 10 Gew.-% Zirkonium enthaltenden Zirkoniumlegierung aufgebaut sind. Die 5-(2-Methylmercaptoethyl)-hydantoin-Lösung wird vorteilhaft am Kopf der Kolonne in der Geschwindigkeit kontinuierlich zugegeben, daß das Hydrolyseprodukt, Kaliummethioninatlösung, am Boden entsprechend abgezogen werden kann; d. h., die Hydrolyse am Boden der Kolonne quantitativ abgelaufen ist. Die gasförmigen Bestandteile, Wasserdampf, Ammoniak und Kohlendioxid, werden günstigerweise am Kopf der Kolonne ausgeschleust und können vorteilhaft zur Wiederherstellung der wässrigen Ammoniak/Kohlendioxid-Lösung zur Herstellung des 5-(2-Methylmercaptoethyl)-hydantoins eingesetzt werden.

Erfindungsgemäß wird zur Hydrolyse des 5-(2-Methylmercaptoethyl)-hydantoins eine wäßrige Lösung aus Kaliumhydroxid, Kaliumcarbonat und/oder Kaliumhydrogencarbonat mit einem Kaliumionengehalt von vorteilhaft 100 - 200 g, vorzugsweise 140 - 160 g, Kalium pro Liter Hydrolyselösung eingesetzt, wobei die aus Zirkonium bestehenden Apparatewandungen und Einbauten einen günstigen katalytischen Effekt auf die Hydrolyse ausüben, so daß diese weitgehend ohne Nebenproduktbildung abläuft. Zweckmäßigerweise wird an dieser Stelle bei einem kontinuierlichen Prozeß die Mutterlauge nach der Methionin-Feststoffabtrennung wieder eingesetzt; die Mutterlauge kann dann zusätzlich noch - der Löslichkeit entsprechend - Rest-Methionin enthalten, auch dies hat sich als günstig für das Verfahren herausgestellt.

Die mittlere Verweilzeit der Reaktionslösung in der Hydrolysekolonne beträgt vorteilhaft 10 - 20 Minuten. Das Molverhältnis der Kaliumionenmenge zu der Summe aus 5-(2-Methylmercaptoethyl)-hydantoin + Methionin beträgt günstig bis zu 10, vorzugsweise 1,3 - 5, insbesondere 1,5 - 2. Die erhaltene Ausbeute an Kaliummethioninat in der Reaktionslösung beträgt bei dieser Verfahrensweise typisch 99.0 - 100 %. Die Konzentration an Kaliummethioninat kann durch entsprechende Auswahl der Hydantoinkonzentration oder durch Verdünnen bzw. Einengen der nach der Hydrolyse anfallenden Lösung eingestellt werden.

Zur vorliegenden Erfindung gehört auch die Freisetzung von Methionin aus Alkalimethioninat, vorteilhaft aus einer wässrigen Lösung, wie sie nach den obigen Verfahrensweisen erhältlich ist. Die Erfindung umfaßt daher auch ein Verfahren zur Herstellung von Methionin aus erfindungsgemäß hergestellten Alkalimethioninat in wässriger Lösung durch Freisetzung mit Kohlendioxid, wobei man der wässrigen Lösung, enthaltend das Alkalimethioninat, vor der Freisetzung des Methionins einen Entschäumer zugibt. Die Erfindung umfaßt außerdem ein Verfahren zur Herstellung von Methionin aus Alkalimethioninat in wässriger Lösung durch Freisetzung mit Kohlendioxid, wobei man die Freisetzung in einem Rührzellenreaktor mit intensiver Durchmischung oder in einem Rührreaktor mit quasi idealer Durchmischung durchführt.

Diese beiden Verfahrensweisen werden vorzugsweise miteinander kombiniert. Als Entschäumer eignen sich alle Verbindungen, die eine schaumbremsende Funktion ausüben. Vorzugsweise wird der Entschäumer in Form einer Dispersion in die Lösung eingebracht. Hierdurch erhält man eine besonders gute Verteilung in der Lösung und nicht eine Konzentrierung im wesentlichen an der Oberfläche der wässrigen Lösung. Hierdurch wird der günstige Effekt des Entschäumers auf die Freisetzung des Methionins, insbesondere die Vermeidung der Bildung von dünnen Plättchen oder Schuppen, begünstigt. Es entstehen feste kugelige Kristalle, überwiegend mit einem Durchmesser von 100 bis 200 µm. Vorteilhaft wird der Entschäumer in einer Konzentration von 1 000 bis 10 000 ppm, bezogen auf das Gesamtmethionin (Methionin + Methioninat, umgerechnet auf Methionin) zugegeben.

Bei der Freisetzung des Methionins aus der wässrigen Lösung mittels Kohlendioxid ist es besonders vorteilhaft, wenn das Kohlendioxid über eine Düsenvorrichtung im Bereich des Bodens in die wäßrige Lösung eingeleitet wird. Dies begünstigt wiederum die Freisetzung des Methionins. Außerdem führt man die Freisetzung vorteilhaft bei einem Druck von 1 bis 30 bar durch, vorzugsweise auch bei einer Temperatur von 0 bis 100 °C.

Ganz besonders vorteilhaft wird eine wäßrige Lösung eingesetzt, die im wesentlichen frei von Ammoniak ist.

Auch die letzte Verfahrensweise wird besonders günstig kontinuierlich durchgeführt. Vorzugsweise wird die beschriebene Verfahrensweise mit der vorher beschriebenen Verfahrensweise zur Herstellung eines Alkalisalzes von Methionin kombiniert, wobei besonders bevorzugt die gesamte Kombination der oben beschriebenen Verfahrensweisen möglich ist.

Im folgenden wird das Verfahren zur Freisetzung von Methionin anhand des bevorzugten Kalium-D,L-Methioninats beschrieben, wobei auch andere Alkali, salze z. B. der Natrium salz möglich sind. Die hierbei angegebenen weiteren bevorzugten oder allgemeinen Verfahrensbedingungen gelten entsprechend auch bei der oben beschriebenen allgemeinen Verfahrensweise.

Bei der Freisetzung von D,L-Methionin aus Kalium-D,L-Methioninat durch Einleiten von Kohlendioxid insbesondere in eine Hydrolyse-Lösung des 5-(2-Methylmercaptoethyl)-hydantoins ist es besonders vorteilhaft, wenn die Lösung praktisch frei von Ammoniak ist. Vorzugsweise enthält die Lösung außerdem gelöstes D,L-Methionin. Außerdem können noch gewisse Mengen an Kaliumcarbonat und Kaliumhydrogencarbonat zugegen sein. Die Lösung kann, sofern gewünscht, vor der Kohlendioxid-Zugabe über Aktivkohle gereinigt werden. Die Kohlendioxid-Zugabe erfolgt üblicherweise bei einer Temperatur von 0 bis 100 °C, vorzugsweise bei 20 bis 35 °C, und üblicherweise bei einem Druck von 1 bis 30 bar, vorzugsweise von 2 bis 5 bar. Vorzugsweise wird das Kohlendioxid so lange in das Reaktionsgemisch eingeleitet, bis ein pH-Wert von 7 bis 9, vorzugsweise 7,5 bis 8,5, erreicht wird und/oder bis die Ausfällung des D,L-Methionins beendet ist. Hierbei ist es besonders vorteilhaft, wenn das Kohlendioxid am Boden eines Reaktors direkt oder zweckmäßigerweise fein verteilt über eine Düsenvorrichtung eingeführt wird. Der Reaktor ist vorteilhaft als Rührzellenreaktor oder als quasi idealer Rührreaktor ausgebildet. Außerdem kann insbesondere bei einer kontinuierlichen Verfahrensweise der Entschäumer zusätzlich noch den Durchsatz erhöhen. Der Entschäumer wird üblicherweise in einer Menge von mindestens 1 000 und zweckmäßig bis 10 000 ppm, vorzugsweise 3 000 bis 5 000 ppm, bezogen auf das in der Reaktionslösung befindliche Gesamtmethionin, insbesondere als wäßrige Emulsion zudosiert. Das freigesetzte Methionin wird vorteilhaft von der Mutterlauge abgetrennt und ist nach dem Trocknen weitgehend staubarm und zeichnet sich durch gute Fließfähigkeit und ein hohes Schüttgewicht aus. Die Methioninpartikel besitzen überwiegend einen Durchmesser von 100 bis 200 µm. Bei dieser Verfahrensweise beträgt die Ausbeute des isolierten D,L-Methionins üblicherweise 98 bis 100 %. Die nach der D,L-Methionin-Abtrennung, insbesondere Filtration, erhaltene Mutterlauge kann vorteilhaft wieder zur Hydrolyse des 5-(2-Methylmercaptoethyl)-hydantoins, ggf. nach Konzentrierung und/oder Abzug von CO₂, eingesetzt werden.

Die Erfindung wird im folgenden anhand von Figuren und Beispielen näher ausgeführt.

Es zeigen
- Fig. 1: eine Verfahrensskizze zur kontinuierlichen Herstellung von 5-(2-Methylmercaptoethyl)-hydantoin (nicht beansprucht);
- Fig. 2: eine Verfahrensskizze zur kontinuierlichen Hydrolyse von 5-(2-Methylmercaptoethyl)-hydantoin zu Alkalimethioninat, und
- Fig. 3: eine Verfahrensskizze zur kontinuierlichen Freisetzung und Isolierung von D,L-Methionin aus Alkali-D,L-Methioninat.

### Beispiel 1 und 2

### Beispiele zur Herstellung von Kalium-Methioninat-Lösung

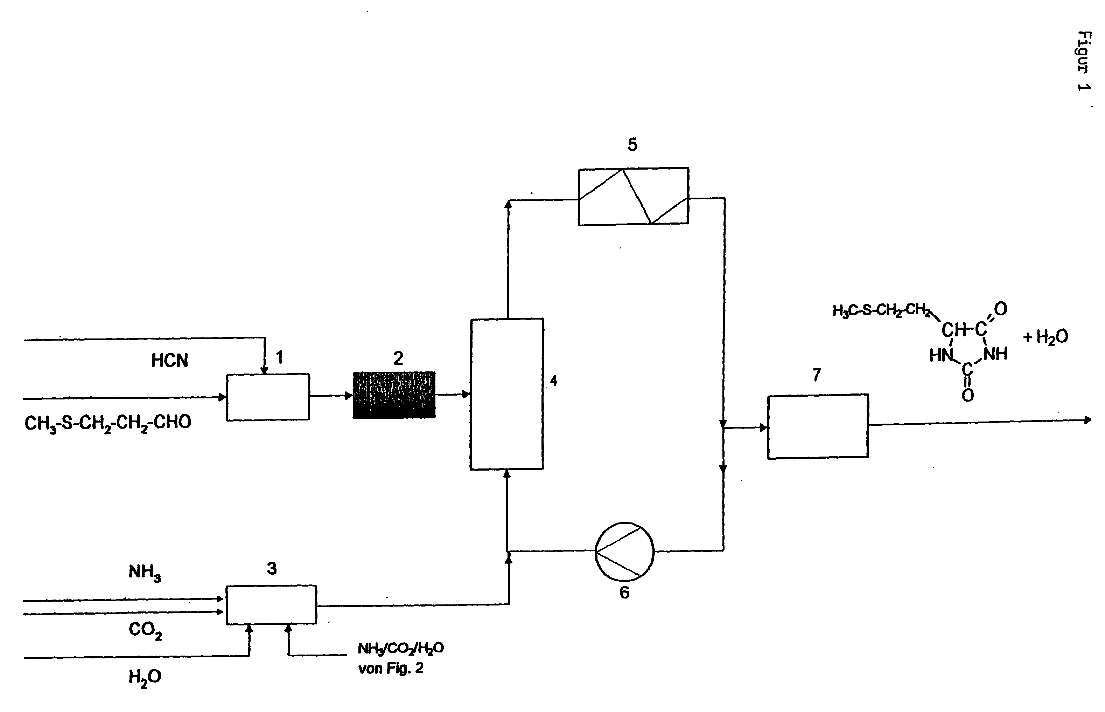

Der allgemeine Verfahrensablauf für die Herstellung von Kalium-Methioninat-Lösung ist in Fig. 1 wiedergegeben.

### Beispiel 1:

### Vergleichsbeispiel mit einer Druckapparatur ohne Zirkoneinbauten

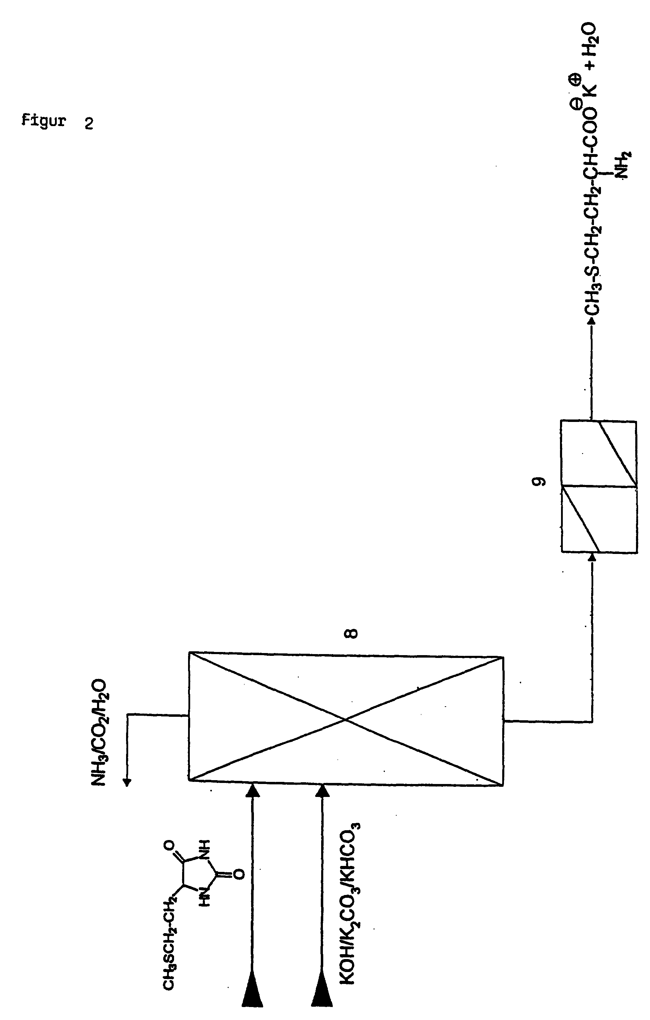

Mittels Pumpendruck wird in eine kontinuierlich arbeitende Druckkolonne 8 aus Edelstahl (vgl. Fig. 2), die mit Dampf betrieben wird, stündlich eine Lösung von 100 kg Kaliumhydrogencarbonat in wäßrige Lösung und 41 kg 5-(2-Methylmercaptoethyl)-hydantoin in 400 1 Wasser eingespeist. Das Reaktionsgemisch wird dabei auf 180 °C aufgeheizt und hat eine mittlere Verweilzeit von ca. 15 min bei etwa 8 bar. Freigesetztes Ammoniak und Kohlendioxid wird am Kopf der Reaktorkolonne über ein Druckhalteventil abgezogen. Am Boden des Druckreaktors wird die Reaktionslösung entspannt und mit dem Wärmetauscher 9 abgekühlt. Dabei werden stündlich 41.9 kg Kalium-Methioninat in Lösung gewonnen (94.5 % d. Th.).

### Beispiel 2:

Es wird eine Apparatur gemäß Fig. 2 verwendet, die eine Druckkolonne beinhaltet, welche Einbauten aus Zirkon besitzt.

Mit einer Pumpe werden stündlich 553 kg 5-(2-Methylmercaptoethyl)-hydantoin in 1600 1 Reaktionslösung aus der Hydantoinherstellung nach Abb. 1 und 3550 l eines Gemisches aus Kaliumcarbonat/Kaliumhydrogencarbonat und Kaliumhydroxid in wässriger Lösung aus der Mutterlaugenrückführung nach der Methioninfeststoffabtrennung mit einem Kaliumgehalt von 140 g pro Liter und einem Rest-Methioningehalt von 120 g pro Liter auf den Kopf einer Druckhydrolysekolonne 8 mit Zirkoneinbauten eingespeist. Die Reaktionstemperatur beträgt 165 °C; der Reaktionsdruck beträgt 7 bar. Das freiwerdende Ammoniak und Kohlendioxid werden am Kopf der Kolonne über ein Druckhalteventil ausgeschleust und der 5-(2-Methylmercaptoethyl)-hydantoin-Synthese erneut zugeführt.
Am Sumpf der Druckapparatur werden 5150 l pro Stunde eines wässrigen Gemisches mit 96.5 g Kalium pro Liter und 175 g Methionin pro Liter (entsprechend einer Zunahme von 476 kg/h Methionin im Gesamtsystem) erhalten (Ausbeute: 100 %). Die Reaktionslösung wird über einen Wärmetauscher 9 abgekühlt und der Methionin-Freisetzung zugeführt.

### Beispiel 3

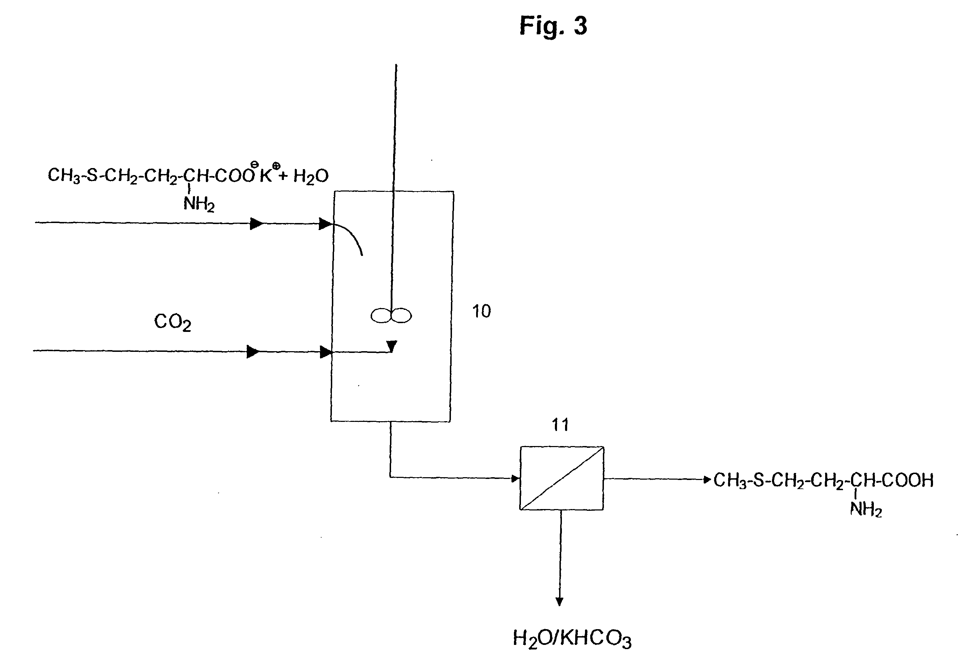

### Beispiel zur Freisetzung von D,L-Methionin aus Kalium-D,Lmethioninat (Fig. 3)

Am Kopf eines Rührreaktors 10 von 340 1 Fassungsvermögen werden kontinuierlich 686 l pro Stunde einer wässrigen Lösung mit 83,6 kg Kalium-D,L-methioninat (Hydrolyselösung von 5-(2-Methylmercaptoethyl)-hydantoin mit zusätzlich 39,77 kg Kreislauf-Methionin und Kaliumverbindungen eingespeist. Gleichzeitig wird am Reaktorboden Kohlendioxid zugeführt, so daß im Reaktor ein Druck von 2 - 3 bar herrscht. Ebenso werden 0,38 kg pro Stunde Entschäumer in Form einer wässrigen Emulsion in den Reaktor eindosiert; diese Menge entspricht ca. 3940 ppm Entschäumer pro Kilogramm Gesamt-Methionin. Die Reaktionstemperatur wird bei 25 °C gehalten. Um im Reaktor ein konstantes Niveau zu halten, werden am unteren Teil des Reaktors der Zudosierung entsprechende Mengen an Reaktionslösung ausgeschleust. Die Ausschleussuspension wird abfiltriert, wobei stündlich 66,5 kg Fest-D,L-Methionin (als Trockensubstanz gerechnet) erhalten werden und die Mutterlauge mit einem Restgehalt von 39,77 kg D,L-Methionin als Verseifungsagens in die Hydantoin-Hydrolysestufe rezykliert werden kann. Die Ausbeute ist quantitativ.

## Patentansprüche

1. Verfahren zur Herstellung eines Alkalisalzes von Methionin durch Hydrolyse von 5-(2-Methylmercaptoethyl)-hydantoin in Gegenwart einer wäßrigen Lösung enthaltend Alkali und Kohlendioxid und ggf. dessen weitere Umsetzung zum Methionin,
**dadurch gekennzeichnet,**
**daß** die Hydrolyse in Apparaturen mit Einbauten aus metallischem Zirkonium oder einer Zirkonium-Legierung durchgeführt wird.

2. Verfahren nach Anspruch 1,
**dadurch gekennzeichnet,**
**daß** die Hydrolyse in einer mit Dampf beheizten Kolonne mit Einbauten durchgeführt wird, bei der die Innenwand und die Einbauten aus Zirkonium oder einer mindestens 10 Gew.-% Zirkonium enthaltenden Zirkoniumlegierung aufgebaut sind.

3. Verfahren nach Anspruch 2,
**dadurch gekennzeichnet,**
**daß** die Hydrolyse zumindest zu Beginn in Gegenwart von mind. 0,1 eq, insbesondere bis 7 eq, Ammoniak je Equivalent 5-(2-Methylmercaptoethyl)-hydantoin durchgeführt wird.

4. Verfahren nach einem der Ansprüche 1 bis 3,
**dadurch gekennzeichnet,**
**daß** die Hydrolyse bei einer Temperatur von 120 bis 250 °C und einem Druck von 5 bis 30 bar durchgeführt wird.

5. Verfahren nach einem der Ansprüche 1 bis 4,
**dadurch gekennzeichnet,**
**daß** zu Beginn der Hydrolyse Methionin in der wässrigen Lösung enthalten ist.

6. Verfahren nach einem der Ansprüche 1 bis 5,
**dadurch gekennzeichnet,**
**daß** als Alkali eine Kaliumverbindung verwendet wird.

7. Verfahren nach einem der Ansprüche 1 bis 6,
**dadurch gekennzeichnet,**
**daß** man die Alkalikomponente equimolar oder im Überschuß bezogen auf das 5-(2-Methylmercaptoethyl)-hydantoin einsetzt.

8. Verfahren nach einem der Ansprüche 5 und 6,
**dadurch gekennzeichnet,**
**daß** das Molverhältnis der Kaliummenge zu der Summe aus 5-(2-Methylmercaptoethyl)-hydantoin + Methionin 1,3 - 5, insbesondere 1,5 - 2 beträgt.

9. Verfahren nach einem der Ansprüche 1 bis 8,
**dadurch gekennzeichnet,**
**daß** man während der Hydrolyse Ammoniak und/oder Kohlendioxid sowie ggf. Wasser aus dem Reaktionssystem ausschleust.

10. Verfahren nach Anspruch 9,
**dadurch gekennzeichnet,**
**daß** das Hydrolysat im wesentlichen frei von Ammoniak und Kohlendioxid entnommen wird.

11. Verfahren nach einem der Ansprüche 1 bis 10,
**dadurch gekennzeichnet,**
**daß** das Verfahren kontinuierlich durchgeführt wird.

12. Verfahren gemäß einem der Ansprüche 1 bis 11,
**dadurch gekennzeichnet,**
**daß** man in eine Alkali-D,L-Methioninat enthaltende Lösung Kohlendioxid bei einer Temperatur von 0 bis 100°C und einem Druck von 1 bis 30 bar einleitet, bis die Ausfällung des D,L-Methionin beendet ist.

13. Verfahren gemäß Anspruch 12,
**dadurch gekennzeichnet,**
**daß** man das Kohlendioxid so lange in das Reaktionsgemisch einleitet, bis ein pH-Wert von 7 bis 9 erreicht wird.

14. Verfahren gemäß den Ansprüchen 12 oder 13,
**dadurch gekennzeichnet,**
**daß** man der Methioninat enthaltenden Lösung vor der Freisetzung des Methionins Entschäumer zusetzt.

15. Verfahren gemäß den Anprüchen 12 bis 14,
**dadurch gekennzeichnet,**
**daß** man die Freisetzung des Methionins in einem Rührzellenreaktor mit intensiver Durchmischung oder ein einem Rührreaktor mit quasi idealer Durchmischung durchführt.

16. Verfahren gemäß Anspruch 11,
**dadurch gekennzeichnet,**
**daß** man nach der Methioninfeststoffabtrennung die Mutterlauge in der Hydrolysestufe einsetzt.

## Claims

1. Process for the preparation of an alkali salt of methionine by hydrolysis of 5-(2-methylmercaptoethyl)-hydantoin in the presence of an aqueous solution containing alkali and carbon dioxide and, optionally, its further reaction to methionine;
**characterised in that**
the hydrolysis is carried out in apparatuses having built-in elements of zirconium metal or of a zirconium alloy.

2. Process according to claim 1,
**characterised in that**
the hydrolysis is carried out in a steam-heated column having built-in elements, in which the inside wall and the built-in elements are made of zirconium or of a zirconium alloy containing at least 10 wt.% zirconium.

3. Process according to claim 2,
**characterised in that**
the hydrolysis is carried out, at least at the beginning, in the presence of at least 0.1 eq., especially up to 7 eq., of ammonia per equivalent of 5-(2-methylmercaptoethyl)-hydantoin.

4. Process according to any one of claims 1 to 3,
**characterised in that**
the hydrolysis is carried out at a temperature of from 120 to 250°C and a pressure of from 5 to 30 bar.

5. Process according to any one of claims 1 to 4,
**characterised in that**
methionine is contained in the aqueous solution at the beginning of the hydrolysis.

6. Process according to any one of claims 1 to 5,
**characterised in that**
a potassium compound is used as the alkali.

7. Process according to any one of claims 1 to 6,
**characterised in that**
the alkali component is used in an equimolar amount or in excess based on the 5-(2-methylmercaptoethyl)-hydantoin.

8. Process according to any one of claims 5 and 6,
**characterised in that**
the molar ratio of the amount of potassium to the sum of 5-(2-methylmercaptoethyl)-hydantoin + methionine is from 1.3 to 5, especially from 1.5 to 2.

9. Process according to any one of claims 1 to 8,
**characterised in that**
ammonia and/or carbon dioxide and, optionally, water are discharged from the reaction system during the hydrolysis.

10. Process according to claim 9,
**characterised in that**
the hydrolysate is removed substantially free of ammonia and carbon dioxide.

11. Process according to any one of claims 1 to 10,
**characterised in that**
the process is carried out continuously.

12. Process according to any one of claims 1 to 11,
**characterised in that**
carbon dioxide is passed at a temperature of from 0 to 100°C and a pressure of from 1 to 30 bar into a solution containing alkali D,L-methioninate until the precipitation of D,L-methionine has ceased.

13. Process according to claim 12,
**characterised in that**
the carbon dioxide is passed into the reaction mixture until a pH value of from 7 to 9 is reached.

14. Process according to claims 12 or 13,
**characterised in that**
anti-foam is added to the methioninate-containing solution before methionine is freed.

15. Process according to claims 12 to 14,
**characterised in that**
the freeing of methionine is carried out in a stirrer cell reactor with intensive thorough mixing or a stirrer reactor with virtually ideal thorough mixing.

16. Process according to claim 11,
**characterised in that**
the mother liquor is used in the hydrolysis step once the methionine solid has been separated off.

## Revendications

1. Procédé de préparation d'un sel alcalin de méthionine par hydrolyse de 5-(2-méthylmercaptoéthyl)hydantoïne en présence d'une solution aqueuse contenant des alcalis et du dioxyde de carbone et, le cas échéant, sa transformation ultérieure en méthionine, **caractérisé en ce que** l'hydrolyse est réalisée dans des appareils avec des structures internes en zirconium métallique ou en alliage de zirconium.

2. Procédé selon la revendication 1, **caractérisé en ce que** l'hydrolyse est réalisée dans une colonne chauffée à la vapeur contenant des structures internes, dans laquelle la paroi intérieure et les structures internes sont réalisées à partir de zirconium ou d'un alliage de zirconium contenant au moins 10 % en poids de zirconium.

3. Procédé selon la revendication 2, **caractérisé en ce que** l'hydrolyse, du moins au début, est réalisée en présence d'au moins 0,1 équivalent, en particulier jusqu'à 7 équivalents, d'ammoniac par équivalent de 5-(2-méthylmercaptoéthyl)hydantoïne

4. Procédé selon l'une quelconque des revendications 1 à 3, **caractérisé en ce que** l'hydrolyse est réalisée à une température de 120 à 250°C et à une pression de 5 à 30 bars.

5. Procédé selon l'une quelconque des revendications 1 à 4, **caractérisé en ce qu'**au début de l'hydrolyse, la méthionine est contenue dans la solution aqueuse.

6. Procédé selon l'une quelconque des revendications 1 à 5, **caractérisé en ce qu'**on utilise comme alcali un composé du potassium.

7. Procédé selon l'une quelconque des revendications 1 à 6, **caractérisé en ce qu'**on utilise les composants alcalins en une quantité équimolaire ou en excès par rapport à la 5-(2-méthylmercaptoéthyl)hydantoïne

8. Procédé selon l'une quelconque des revendications 5 et 6, **caractérisé en ce que** le rapport molaire de la quantité de potassium à la somme de 5-(2-méthylmercaptoéthyl)hydantoïne et de méthionine est de 1,3 à 5, en particulier de 1,5 à 5.

9. Procédé selon l'une quelconque des revendications 1 à 8, **caractérisé en ce qu'**on évacue du système de réaction, pendant l'hydrolyse, de l'ammoniac et/ou du dioxyde de carbone ainsi que, le cas échéant, de l'eau.

10. Procédé selon la revendication 9, **caractérisé en ce que** l'hydrolysat est prélevé en étant essentiellement exempt d'ammoniac et de dioxyde de carbone.

11. Procédé selon l'une quelconque des revendications 1 à 10, **caractérisé en ce que** le procédé est réalisé de manière continue.

12. Procédé selon l'une quelconque des revendications 1 à 11, **caractérisé en ce qu'**on introduit dans une solution contenant un D,L-méthionate de métal alcalin du dioxyde de carbone à une température de 0 à 100°C et une pression de 1 à 30 bars, jusqu'à ce que la séparation par précipitation de la D,L-méthionine soit terminée.

13. Procédé selon la revendication 12, **caractérisé en ce qu'**on introduit le dioxyde de carbone aussi longtemps dans le mélange réactionnel jusqu'à ce qu'on atteigne un pH de 7 à 9.

14. Procédé selon l'une quelconque des revendications 12 ou 13, **caractérisé en ce qu'**on ajoute un antimousse à la solution contenant le méthionate, avant la libération de la méthionine.

15. Procédé selon les revendications 12 à 14, **caractérisé en ce qu'**on réalise la libération de la méthionine dans un réacteur à cellule agitée avec un mélange intensif ou un réacteur agité avec un mélange quasi idéal.

16. Procédé selon la revendication 11, **caractérisé en ce qu'**on utilise, après la séparation de la méthionine solide, la lessive-mère dans l'étape d'hydrolyse.
